# EUROPEAN PATENT APPLICATION

(11) **EP 2 754 446 A1**
(43) Date of publication of application: **16.07.2014**
(21) Application number: 13183293.3
(22) Date of filing: 06.09.2013
(51) Int. Cl.: A61K 35/74, A61P 13/00

(54) **A probiotic composition for the treatment of uremic toxins**

(30) Priority: 10.01.2013 TW 102100878
(71) Applicant: National Taiwan University, Taipei 10617 (TW); Conmed Pharmaceutical & Bio-Medical Corporation, Taipei (TW)
(72) Inventor: Chen, Bao-Ji, Taipei 106 (TW); Chen, Ming-Ju, Taipei 106 (TW); Liu, Je-Ruei, Taipei (TW); Fang, Chen-Yu, Taipei (TW)
(74) Representative: Chaillot, Geneviève

(57) **Abstract**

The invention relates to a probiotic composition for reducing uremic toxins and the manufacturing method thereof. The probiotic composition comprises at least one selected from the group consisting of: *Lactobacillus plantarum* BCRC 12251, *Lactobacillus paracasei* BCRC 12188, *Streptococcus thermophilus* BCRC 13869 and pharmaceutically acceptable vehicles,_excipients, diluents, adjuvants, *etc.* The invention further relates to a probiotic composition consisted of *Lactobacillus plantarum* BCRC 12251, *Lactobacillus paracasei* BCRC 12188, *Streptococcus thermophilus* BCRC 13869 and pharmaceutically acceptable vehicles,_excipients, diluents, adjuvants, *etc.* In addition, the invention relates to a novel use of the probiotic compositon for reducing uremic toxins, wherein the uremic toxins are protein-bound uremic toxins.

## Description

### BACKGROUND OF THE INVENTION

### 1. FIELD OF THE INVENTION

The invention provided herein relates to a probiotic composition for the treatment of uremic toxins by reducing such uremic toxins, and the manufacturing method thereof..

### 2. DESCRIPTION OF THE PRIOR ART

Chronic kidney disease (CKD) is a disorder that the kidneys gradually loss their normal function and has become a worldwide health problem. In addition, the disorder also dramatically affects the quality of life in CKD patients. Progressive loss of kidney functions in CKD patients inevitably results in accumulation of wastes that originally was eliminated or metabolized by the kidneys as well as increase of blood concentrations of these wastes, which consequently induces toxicity in various organs *in vivo.* Uremic retention solutes (URMs) found in patients with kidney failure are various molecules, that were normally removed by the healthy kidneys, and are accumulated in blood or tissues in the progression of chronic kidney disease to kidney failure. Since uremic retention solutes contribute to development of uremia, they are also called uremic toxins. URMs, according to characteristics that affect their removal pattern during dialysis, are classified into three groups by European Toxin Work Group: (1) small water-soluble molecules (<500 Da) which can easily pass any dialysis filter; (2) larger molecules **( ≧** 500 Da), sometimes also called middle molecules, are molecules with restricted passage based on the features of the filter; and (3) protein-bound solutes in which the clearance of these solutes through dialysis depends greatly on the balance between bound and free fractions. Additionally, adsorptive techniques may affect the efficacy on reducing the protein-bound solutes.

Classification of the URMS can facilitate the medical field to further understand the features of various uremic toxins and to develop better treatment methods. For a long time, majority of the studies have been focused on the effects of small water-soluble molecules on kidney diseases, or even used these molecules as the basis for evaluation of the treatment results. However, with more research results been published, a new theory has been gradually developed, which is the protein-bound solutes play far more important roles in the development of kidney diseases, and therefore, their effects on treating patients with kidney disease need to be reassessed.

### Protein-bound uremic toxins

Protein-bound uremic toxins is not only associated with uremic syndrome, but may also related to the high mortality rate found in patients with CKD. Thus, numerous researches had devoted in reduction of the plasma concentration of protein-bound uremic toxins, which mainly include two methods: (1) reduction of the absorption of protein-bound uremic toxins in intestine; and (2) improvement of the blood clearance of the protein-bound uremic toxins.

Indoxyl sulfate (IS) and p-cresyl sulfate are two very important protein-bound uremic toxins, and are the most widely used marker molecules in studying the effects of protein-bound uremic toxins on hemodialysis, hemodiafiltration or peritoneal dialysis. Moreover, Indoxyl sulfate (IS) and p-cresyl sulfate are also considered to have direct association with the development of uremic syndrome.

At present, the key method used for removal of the uremic toxins is dialysis which can eliminate uremic toxins that are water-soluble small molecules. Yet, none of these methods can efficiently remove protein-bound uremic toxins from blood. Furthermore, some studies even suggested that dialysis membranes are superior in elimination of water-soluble small molecules, whereas their clearance rates of protein-bound uremic toxins are extremely low.

In addition, Marier *et al.* disclosed a method for treating chronic kidney disease by administration of an oral adsorbent (AST-120, Kremezin, Kureha Corporation, Tokyo, Japan). AST-120 (an activated charcoal adsorbent) was given, which functions in the large intestine and can adsorb different organic compounds, such as indoxyl sulfate and p-cresol, so as to reduce adsorption of the protein-bound uremic toxins by the body. Nonetheless, the oral adsorbent is currently in clinical trial for determination of its most effective and adequate dosages.

In summary, further improvements in traditional methods for removal of uremic toxins are highly desired.

### SUMMARY OF THE INVENTION

To further improve the methods for removal of uremic toxins, the inventor of the present invention has developed a technology for reducing uremic toxins using probiotics, and said uremic toxins, in particular, refer to protein-bound uremic toxins. The present invention provides a probiotic composition for the treatment of various uremic toxins.

In one aspect, the present invention provides an alternative treatment that overcomes the issues faced by other traditional treatments for elimination of uremic toxins, such as inefficient clearance of protein-bound uremic toxins found in hemodialysis.

In the present invention, the probiotic composition for the treatment of uremic toxins comprises at least one selected from the group consisting of: *Lactobacillus plantarum* BCRC 12251, *Lactobacillus paracasei* BCRC 12188, *Streptococcus thermophilus* BCRC 13869 and *Enterococcus faecalis,* and the probiotic composition can be used for removal of blood uremic toxins.

The present invention further discloses a probiotic composition, the Pm-1 probiotic composition (Probiotic mix-1), comprising *Lactobacillus plantarum* BCRC 12251, *Lactobacillus paracasei* BCRC 12188, and *Streptococcus thermophilus* BCRC 13869, wherein the Pm-1 exhibits the best effects in clearance of uremic toxins; wherein, the uremic toxins are protein-bound uremic toxins; furthermore, said protein-bound uremic toxins are indoxyl sulfate, p-cresol or phenol. The concentration of the probiotics in Pm-1 is 10⁷-10¹⁰ CFU/mL; the ratios of various probiotics in Pm-1 are 40-60%, 20-30%, and 20-30% for *Lactobacillus plantarum* BCRC 12251, *Lactobacillus paracasei* BCRC 12188, and *Streptococcus thermophilus* BCRC 13869, respectively.

Although currently other treatments are available for removal of uremic toxins, such as hemodialysis and oral adsorbent (AST-120), hemodialysis is mainly used for elimination of small-molecule compounds and cannot effectively remove protein-bound uremic toxins in blood. On the other hand, oral adsorbents can reduce the absorption of uremic toxins *in vivo,* but it is now under the investigation in clinical trials. Therefore, a certain waiting period is necessary before its actual application in treatment. On the contrary, the Pm-1 probiotic composition disclosed in present invention not only has superior effects on removal of protein-bound uremic toxins, but also is easy to prepare and hence is more economic.

### BRIEF DESCRIPTION OF THE DRAWINGS

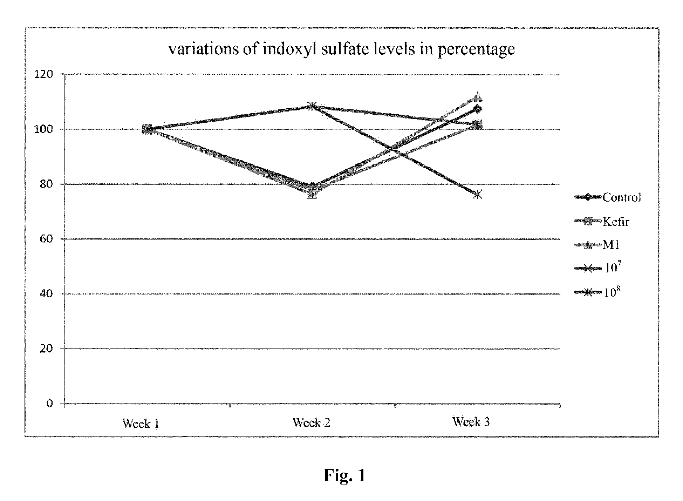

FIG. 1 shows variations of indoxyl sulfate levels in blood collected from the mice given different strains of bacteria (Control; Kefir; M1: *Lb kefiranofaciens* suspension in PBS; 10⁷:mixture of BCRC 12251 10⁷ CFU/m L and BCRC 12188 10⁷ CFU/mL; mixture of BCRC 12251 10⁸ CFU/mL and BCRC 12188 10⁸ CFU/mL).

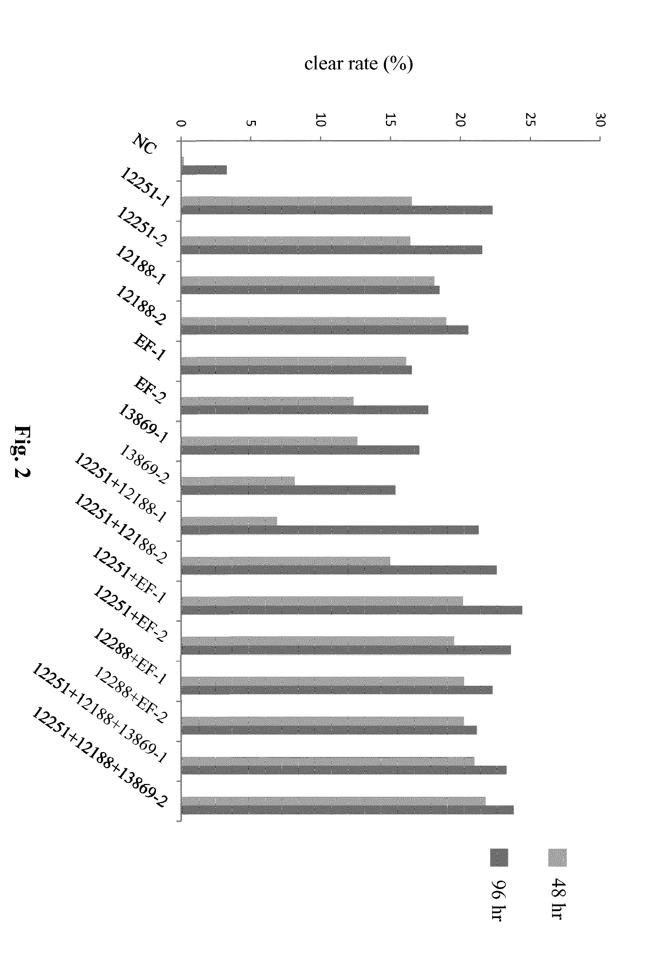

FIG. 2 shows the indoxyl sulfate clearance efficiency of various probiotic compositions including single strains 12188, 12251, 13869 and EF, and mixed strains including 12251+12188, 12251+EF, 12188+EF and 12251+12188+13869.

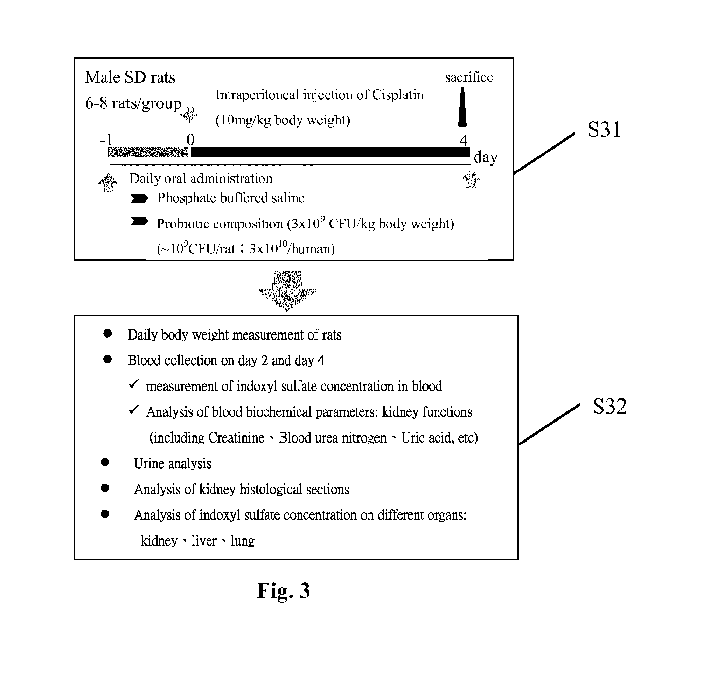

FIG. 3 is the flow chart for animal study of the present invention. S31 is the flow chart for animal study using Cisplatin for induction of acute kidney injury in rats, and S32 demonstrates the method selected for analyzing Cisplatin-induced kidney injury in rats.

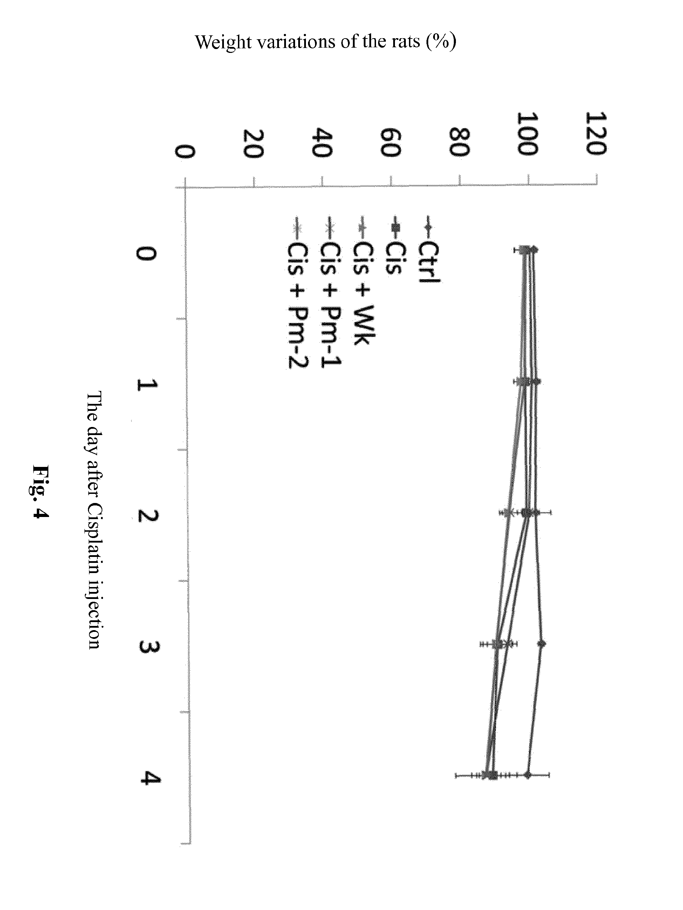

FIG. 4 shows the weight variations of the rats during the study period of the present invention (ctrl: control group, not injected with Cisplatin; Cis: positive control group, injected with Cisplatin but no probiotics treatment; Cis+Wk: injected with Cisplatin and received Wk (Wakamoto) treatment; Cis+Pm-1: injected with Cisplatin and received probiotic mix-1 treatment; Cis+Pm-2: injected with Cisplatin and received probiotic mix-2 treatment) .

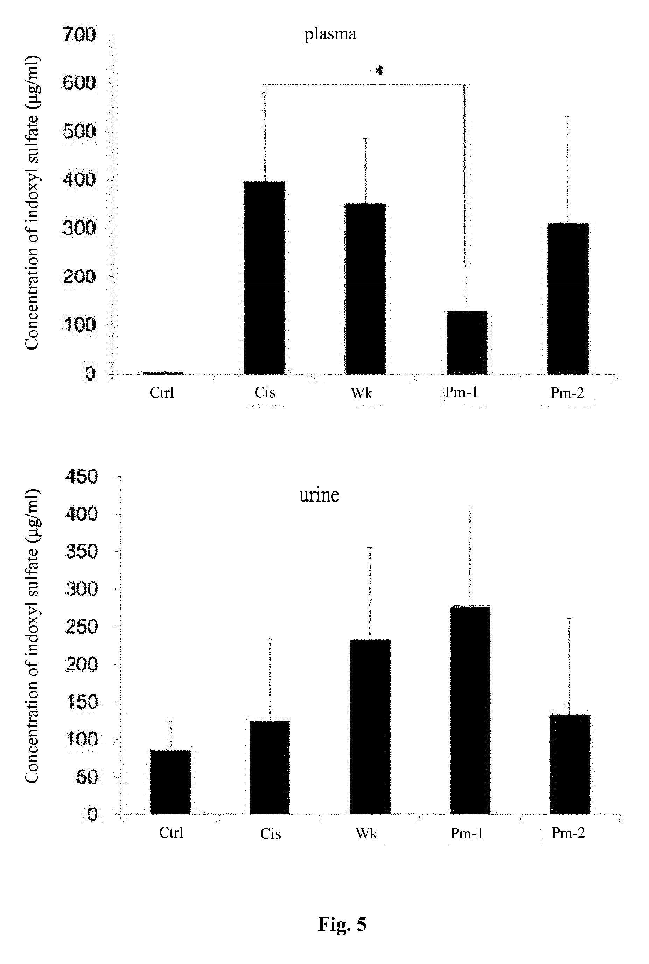

FIG. 5 shows indoxyl sulfate levels in plasma and urine collected from rats with Cisplatin-induced acute kidney injury (ctrl: control group, not injected with Cisplatin; Cis: positive control group, injected with Cisplatin but received no probiotics treatment; Cis+Wk: injected with Cisplatin and received Wk treatment; Cis+Pm-1: injected with Cisplatin and received probiotic mix-1 treatment; Cis+Pm-2: injected with Cisplatin and received probiotic mix-2 treatment).

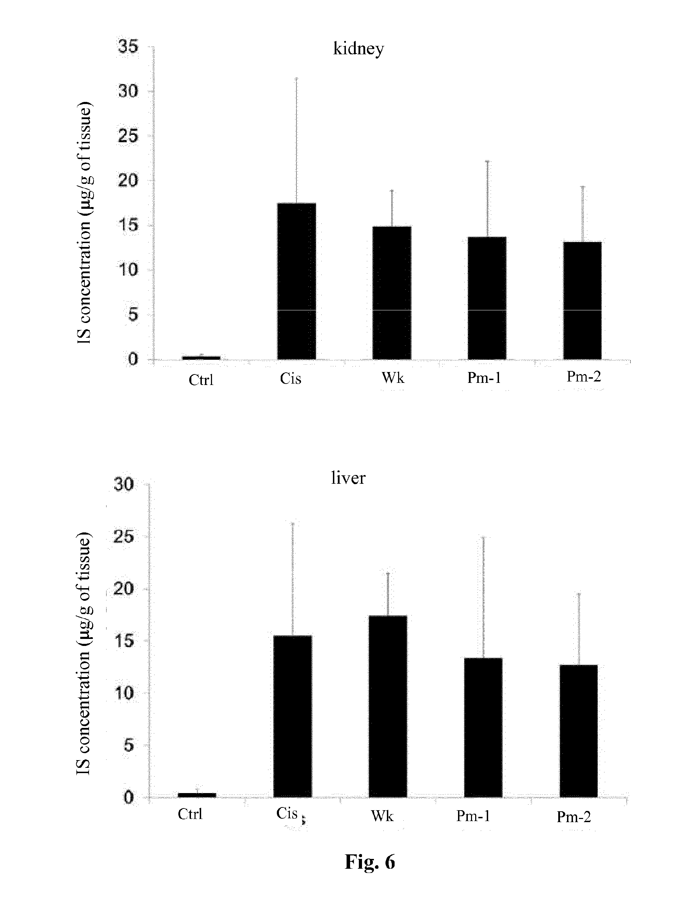

FIG. 6 shows indoxyl sulfate levels in kidney and liver collected from rats with Cisplatin-induced kidney injury (ctrl: control group, not injected with Cisplatin; Cis: positive control group, injected with Cisplatin but received no probiotics treatment; Cis+Wk: injected with Cisplatin and received Wk treatment; Cis+Pm-1: injected with Cisplatin and received probiotic mix-1 treatment; Cis+Pm-2: injected with Cisplatin and received probiotic mix-2 treatment).

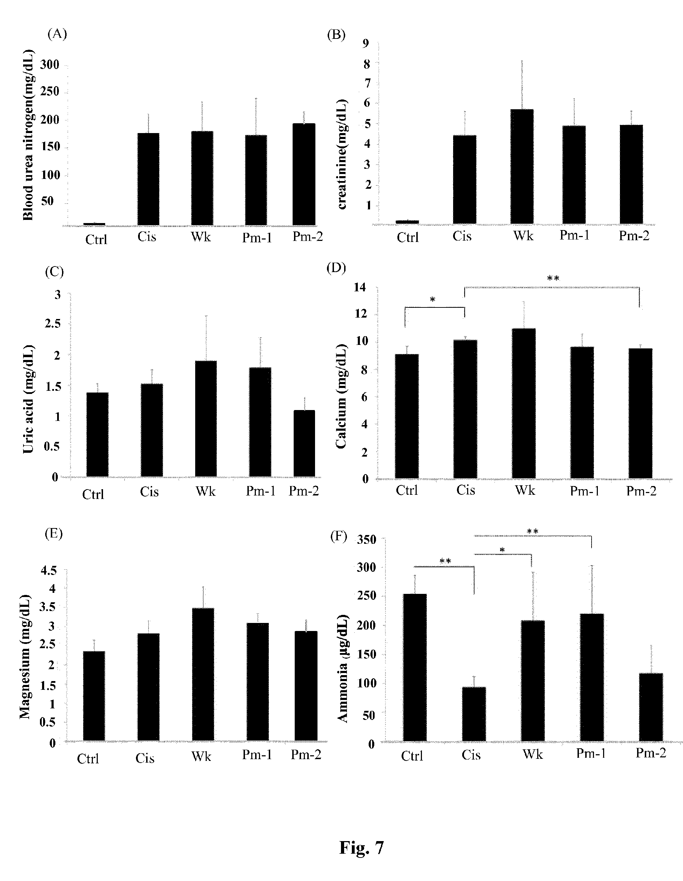

FIG. 7 shows the results of blood biochemical analysis of rats with Cisplatin-induced acute kidney injury (*p<0.05, **p<0.01, ctrl: control group, not injected with Cisplatin; Cis: positive control group, injected with Cisplatin but received no probiotics treatment; Cis+Wk: injected with Cisplatin and received Wk treatment; Cis+Pm-1: injected with Cisplatin and received probiotic mix-1 treatment; Cis+Pm-2: injected with Cisplatin and received probiotic mix-2 treatment).

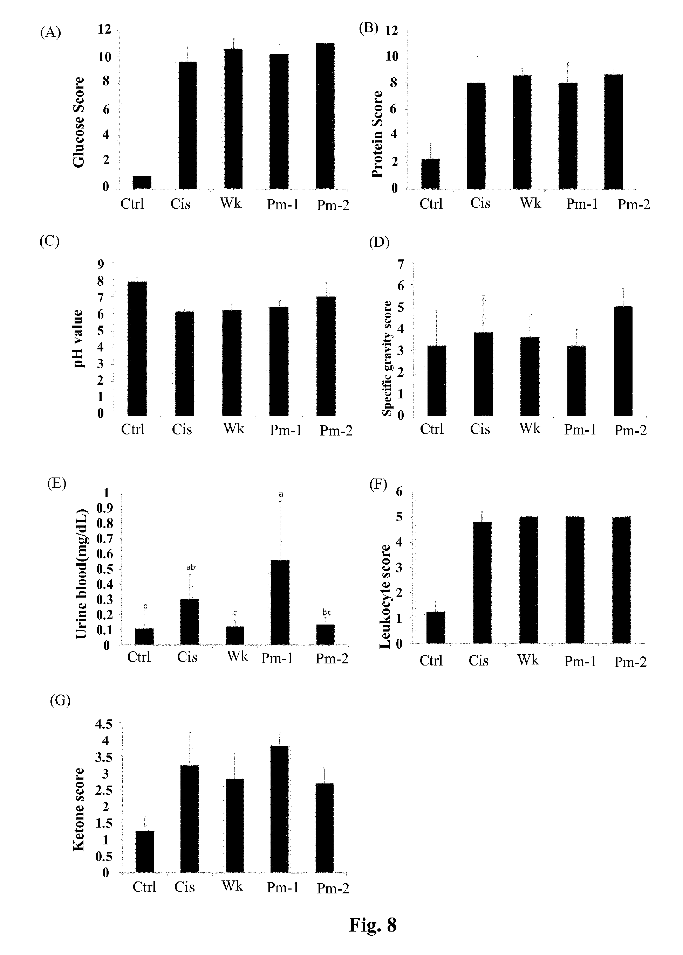

FIG. 8 shows the results of urine biochemical analysis results of rats with Cisplatin-induced acute kidney injury (ctrl: control group, not injected with Cisplatin; Cis: positive control group, injected with Cisplatin but received no probiotics treatment; Cis+Wk: injected with Cisplatin and received Wk treatment; Cis+Pm-1: injected with Cisplatin and received probiotic mix-1 treatment; Cis+Pm-2: injected with Cisplatin and received probiotic mix-2 treatment. Wherein, in Figure 8E, various symbols are used including c, ab, c, a, and bc, and groups labeled with the same symbol indicate that no statistical differences was found.)

### DETAILED DESCRIPTION OF THE PREFERRED EMBODIMENT

The invention will now be further illustrated by the following examples. However, it should be noted that the scope of present invention is not limited by the examples provided herein.

### Example 1

In the example 1 of the invention, a probiotic bacteria strain is provided by screening of probiotics that exhibit better clearance rates for removal of uremic toxins:

### 1. The process of probiotics selection:

(1) An aliquot of 100 µL activated bacteria culture was inoculated into culture media containing different uremic toxins and samples were collected at 0hr, 24hr, and 48hr, wherein the concentrations of the uremic toxins in the culture media are 60 µg/ml indoxyl sulfate, 200µg/ml phenol and 500 µg/ml p-cresol.
(2) The collected samples were subjected to HPLC (High performance liquid chromatography) analysis for their uremic toxins clearance rates.
(3) The analyzed uremic toxins were indoxyl sulfate, p-cresol or phenol.

### 2. Results:

### (1) The clearance rate of indoxyl sulfate (IS)

The clearance rates of IS obtained from various probiotic stains are analyzed by HPLC and then converted into percentages (as shown in Table 1), and the results indicated that all probiotic strains can decrease IS levels in all the samples collected. Among which, strain no. 12251 shows the most significant reduction with a clearance rate of 19.45% at 48hr, followed by strains no.14039 and no.12195 with a clearance rate of 19.18% and 18.6%, respectively.

**Table 1 The clearance rates of indoxyl sulfate (IS)**

| | Clearance rate (%) | | | Clearance rate (%) | |
|---|---|---|---|---|---|
| **Strains*** | 24 hr | 48hr | **Strains** | 24 hr | 48 hr |
| **12251** | 12.41 | 19.45 | **12586** | 2.42 | 11.50 |
| **14039** | 16.94 | 19.18 | **10695** | 6.03 | 11.17 |
| **12195** | 9.03 | 18.60 | **14079** | 3.13 | 10.82 |
| **12936** | 9.16 | 17.61 | **14669** | 5.70 | 10.54 |
| **14008** | 8.65 | 17.05 | **10696** | -0.03 | 10.42 |
| **12263** | 11.66 | 16.80 | **14667** | 2.39 | 9.86 |
| **11846** | 9.38 | 15.69 | **14668** | 4.70 | 9.63 |
| **14660** | 10.52 | 15.39 | **12272** | 2.70 | 9.61 |
| **14620** | 4.75 | 15.21 | **14628** | -0.83 | 8.48 |
| **14666** | 5.99 | 15.15 | **14622** | 0.57 | 8.26 |
| **10361** | 9.10 | 14.72 | **14011** | 1.88 | 6.60 |
| **16000** | 6.17 | 13.92 | **12187** | 1.75 | 4.45 |
| **12188** | 5.23 | 13.72 | **12247** | 1.67 | 4.35 |
| **17394** | 7.65 | 13.29 | **14023** | 0.38 | 3.30 |
| **10069** | 2.26 | 13.19 | **10940** | 0.28 | 2.35 |
| **17638** | 2.34 | 12.81 | **10697** | 0.27 | 2.23 |
| **14615** | 6.44 | 12.03 | | | |

| | | | | | |
|---|---|---|---|---|---|
| Strain*: Probiotic strains were purchased from Bioresource Collection and Research Center (BCRC) (a total of 33 strains including 10069, 10361, 10695, 10696, 10697, 10940, 11846, 12187, 12188, 12195, 12247,12251,12263,12272,12586,12936,14008,14011,14023,14039, 14079,14615,14620,14622,14628,14660,14666,14667,14668,14669, 16000, 17394, 17638 were obtained) and cultured in Lactobacilli MRS media (Difco Laboratories, Detroit, MD) to activate the bacteria. | | | | | |

### (2) The clearance rate of p-cresol

The collected samples were analyzed by HPLC and then converted into percentages, and the clearance rates of p-cresol of each strain are shown in Table 2. The clearance rates at 48hr post inoculation are showed from the highest to the lowest: strain 14615, 14666 and 12188, and all three strains have a clearance rate of p-cresol above 4%.

**Table 2 The clearance rates of p-cresol**

| | Clearance rate (%) | | | Clearance rate (%) | |
|---|---|---|---|---|---|
| **Strain** | 24 hr | 48 hr | **Strain** | 24 hr | 48 hr |
| **14615** | 3.61 | 4.57 | **10069** | 1.11 | 2.48 |
| **14666** | 0.79 | 4.44 | **14660** | 2.20 | 2.47 |
| **12188** | 3.63 | 4.03 | **10695** | 0.66 | 2.28 |
| **14668** | 1.80 | 3.80 | **14628** | 1.54 | 2.23 |
| **10697** | 3.13 | 3.72 | **10361** | 2.06 | 2.06 |
| **12247** | 1.49 | 3.64 | **12263** | 0.29 | 1.93 |
| **14667** | 3.14 | 3.56 | **14622** | 1.06 | 1.70 |
| **12251** | -2.46 | 3.54 | **14079** | -3.28 | 1.35 |
| **17394** | -0.53 | 3.52 | **12272** | -0.15 | 0.93 |
| **16000** | 2.60 | 3.48 | **12195** | 0.75 | 0.88 |
| **10696** | -0.23 | 3.41 | **12586** | 0.76 | 0.86 |
| **14669** | 3.75 | 3.39 | **10940** | 0.40 | 0.73 |
| **14620** | 2.21 | 3.03 | **17638** | 0.35 | 0.72 |
| **11846** | 1.15 | 2.85 | **14039** | 0.39 | 0.70 |
| **12936** | 2.13 | 2.82 | **12187** | -6.15 | 0.62 |
| **14023** | 2.16 | 2.56 | **14008** | 2.62 | -3.71 |
| **14011** | 0.46 | 2.54 | | | |

### (3) The clearance rate of phenol

The collected samples were analyzed by HPLC and then converted into percentages, and the clearance rates of phenol of each strain are shown in Table 3. Strains 12188, 12187 and 12247 are the three strains with highest clearance rates at 48hr post inoculation, and the clearance rates are 6.83%, 6.37% and 4.96%, respectively.

**Table 3 The clearance rates of phenol**

| | Clearance rate (%) | | | Clearance rate (%) | |
|---|---|---|---|---|---|
| **Strain** | 24 hr | 48 hr | **Strain** | 24 hr | 48 hr |
| **12188** | -2.63 | 6.83 | **12272** | 2.18 | 1.36 |
| **12187** | 2.28 | 6.37 | **10069** | 0.12 | 1.26 |
| **12247** | 2.43 | 4.96 | **17394** | -1.11 | 0.84 |
| **14615** | 3.61 | 4.57 | **11846** | 2.85 | 0.70 |
| **14666** | 0.84 | 4.07 | **14039** | 1.74 | 0.60 |
| **12586** | -4.13 | 3.98 | **14079** | 2.68 | 0.18 |
| **14669** | 2.71 | 3.92 | **14008** | 5.40 | -0.78 |
| **14622** | 3.07 | 3.52 | **17638** | 2.85 | -0.80 |
| **14620** | 4.23 | 3.22 | **12251** | 2.83 | -1.43 |
| **10696** | 1.39 | 3.19 | **12263** | -3.81 | -1.50 |
| **14011** | 1.61 | 3.01 | **14660** | 0.95 | -1.80 |
| **14667** | 2.12 | 2.41 | **16000** | 1.60 | -2.60 |
| **10361** | 3.93 | 1.98 | **12936** | 1.72 | -2.61 |
| **10697** | 1.98 | 1.91 | **14023** | 1.99 | -2.90 |
| **10695** | 0.13 | 1.74 | **14668** | 2.03 | -3.20 |
| **12195** | 1.23 | 1.70 | **14628** | 0.02 | -3.90 |
| **10940** | -0.01 | 1.61 | | | |

**Table 4 Comparison of the clearance rates of indoxyl sulfate, p-cresol and phenol.**

| **Strain** | **Clearance rate (%)** | | | | | |
|---|---|---|---|---|---|---|
| | Indoxyl sulfate | | p-cresol | | Phenol | |
| | 24 hr | 48 hr | 24 hr | 48 hr | 24 hr | 48 hr |
| **10069** | 2.26 | 13.19 | 1.11 | 2.48 | 0.12 | 1.26 |
| **10361** | 9.10 | 14.72 | 2.06 | 2.06 | 3.93 | 1.98 |
| **10695** | 6.03 | 11.17 | 0.66 | 2.28 | 0.13 | 1.74 |
| **10696** | -0.03 | 10.42 | -0.23 | 3.41 | 1.39 | 3.19 |
| **10697** | 0.27 | 2.23 | 3.13 | 3.72 | 1.98 | 1.91 |
| **10940** | 0.28 | 2.35 | 0.40 | 0.73 | -0.01 | 1.61 |
| **11846** | 9.38 | 15.69 | 1.15 | 2.85 | 2.85 | 0.70 |
| **12187** | 1.75 | 4.45 | -6.15 | 0.62 | 2.28 | 6.37 |
| **12188** | 5.23 | 13.72 | 3.36 | 4.03 | -2.63 | 6.83 |
| **12195** | 9.03 | 18.60 | 0.75 | 0.88 | 1.23 | 1.70 |
| **12247** | 1.67 | 4.35 | 1.49 | 3.64 | 2.43 | 4.96 |
| **12251** | 12.41 | 19.45 | -2.46 | 3.54 | 2.83 | -1.43 |
| **12263** | 11.66 | 16.80 | 0.29 | 1.93 | -3.81 | -1.50 |
| **12272** | 2.70 | 9.61 | -0.15 | 0.93 | 2.18 | 1.36 |
| **12586** | 2.42 | 11.50 | 0.76 | 0.86 | -4.13 | 3.98 |
| **12936** | 9.16 | 17.61 | 2.13 | 2.82 | 1.72 | -2.61 |
| **14008** | 8.65 | 17.05 | 2.62 | -3.71 | 5.40 | -0.78 |
| **14011** | 1.88 | 6.60 | 0.46 | 2.54 | 1.61 | 3.01 |
| **14023** | 0.38 | 3.30 | 2.16 | 2.56 | -1.99 | -2.9 |
| **14039** | 16.94 | 19.18 | 0.39 | 0.70 | 1.74 | 0.60 |
| **14079** | 3.13 | 10.82 | -3.28 | 1.35 | 2.68 | 0.18 |
| **14615** | 6.44 | 12.03 | 3.61 | 4.57 | 3.61 | 4.57 |
| **14620** | 4.75 | 15.21 | 2.21 | 3.03 | 4.23 | 3.22 |
| **14622** | 0.57 | 8.26 | 1.06 | 1.70 | 3.07 | 3.52 |
| **14628** | -0.83 | 8.48 | 1.54 | 2.23 | 0.02 | -3.90 |
| **14660** | 10.52 | 15.39 | 2.20 | 2.47 | 0.95 | -1.80 |
| **14666** | 5.99 | 15.15 | 0.79 | 4.44 | 0.84 | 4.07 |
| **14667** | 2.39 | 9.86 | 3.14 | 3.56 | 2.12 | 2.41 |
| **14668** | 4.70 | 9.63 | 1.80 | 3.80 | 2.03 | -3.20 |
| **14669** | 5.70 | 10.54 | 3.75 | 3.39 | 2.71 | 3.92 |
| **16000** | 6.17 | 13.92 | 2.60 | 3.48 | 1.60 | -2.60 |
| **17394** | 7.65 | 13.29 | -0.53 | 3.52 | -1.11 | 0.84 |
| **17638** | 2.34 | 12.81 | 0.35 | 0.72 | 2.85 | -0.80 |
| **Control group** | 2.99 | 4.36 | 2.29 | 0.63 | 1.67 | 1.72 |

### 3. Conclusion:

As indicated in the comparison table, Table 4, amongst the top five strains that have the best clearance effects, strains no. 14615, 14666 and 12188 exhibit better effects on clearing phenol and p-cresol. Likewise, the three strains also demonstrates IS clearance effects with the rate of 12.03%, 15.15% and 13.72%, respectively. However, the strains that have better IS clearance effects all exhibit lower phenol and p-cresol clearance.

In addition, according to Tables 1, 2, and 3, most strains show better clearance effects on indoxyl sulfate than on phenol and p-cresol. The clearance rate of indoxyl sulfate obtained from majority strains are all over 10% at 48 hr post inoculation, some even up to 18∼19%. On the contrary, the best clearance rates obtained for phenol and p-cresol are between 4∼6%, and most strains show only limited clearance effects, around 1∼2%, on both phenol and p-cresol. Hence, we speculated that the significant variations found between various clearance rates may be due to different concentrations of the added uremic toxins.

Because the concentration of indoxyl sulfate added in the above mentioned indoxyl sulfate clearance study is 60 µg/mL, while the concentrations of phenol and p-cresol added in the clearance studies are 200 µg/mL and 500 µg/mL, respectively, which are both significantly higher than 60 µg/mL. Therefore, from the results it may imply the amounts of the uremic toxins which can be removed by probiotics may have limitation, and this limitation is account for the differences observed in the clearance rates for difference uremic toxins.

For the positive control groups that are not inoculated, their concentrations all show certain decrease and may be due to spontaneous volatilization or disassembling of the compounds . However, because the reduced amount is rather limited, the analyzed results of the example are not affected.

Negative clearance rate might be due to sampling or analysis errors since no uremic toxins were detected in the negative control group that contains no uremic toxins. Hence, the negative value cannot be resulted from the toxins produced by the probiotics.

In summary, for mixed probiotics treatments and animal studies conducted afterwards, stains 12251 and 12188 were selected for testing the effectiveness on reducing uremic toxins due to their high efficacies on reducing indoxyl sulfate and phenol and p-cresol, respectively.

### Example 2

In the example 2 of the present invention, the probiotic strains12251 and 12188 which exhibited better uremic toxin clearance in example 1 are selected for preparation of a probiotic composition for clearing uremic toxins.

### 1. Animal study in mice:

### (1) Administration of the probiotic composition

Six-week old BALB/c mice were obtained and fed for 4 weeks before subjected to oral administration of the probiotic composition for 28 days every day at the age of 10-week old. The mice were randomly divided into five groups (8 mice each group): control group and Kefir group were administered with PBS (phosphate buffer saline) and Kefir, respectively. The remaining three groups were given 200 µL of different probiotics suspended in PBS at different concentrations: *Lb. kefiranofaciens* M1 at 1X10⁸ CFU/mL, the mixture of BCRC 12251 and 12188 at 1X10⁷ CFU/mL each, and the mixture of BCRC 12251 and 12188 at 1X10⁸ CFU/mL each.

### (2) Serum sample collection

The serum samples were collected on the day 0, 7, 14, and 21 by cheek-pouch blood collection. The collected blood was centrifuged at 6,000 x g for 90 seconds and the serum was separated and subjected to measurement of indoxyl sulfate concentration.

### (3) Indoxyl sulfate clearance test

On the day 28, the mice were abstained from eating for 20 hrs before orally administered with 5mg/mL indoxyl sulfate in 0.5% methyl cellulose. The final dose, 50 mg/kg, was adjusted according to the body weights of the mice, and blood samples were collected 2 hrs and 8 hrs after giving IS by cheek-pouch blood collection followed by measurement of serum IS concentration.

### (4) Indoxyl sulfate (IS) analysis:

The samples obtained by cheek-pouch blood collection were then subjected to HPLC analysis for indole. CAPCELLPARK MF Ph-1 SG80 column (4.6*150mm) with Guards (4.0*10mm) was used in the present study. The solution used at mobile phase was 50 mM ammonium acetate solution (3.854g/1L) with a flow rate at 1mL/min, and then examined by a fluorescence detector. Standards ranging from 15 to 120µg/mL were prepared by adding indoxyl sulfate to de-ionized water. HPLC analysis were performed according to the above conditions, and the calibration curve was then calculated and used as standards for determining the IS levels in the samples.

### 2. Results:

Blood IS levels of the mice following oral administration with 1X10⁸ CFU/mL mixed bacteria for 3 weeks are significantly reduced (Fig. 1), whereas in other treatment groups as well as the control groups, blood IS levels decreased notably at week 2, but then raised again at week 3. The findings are not observed in the groups treated with the mixed bacteria; thus, reproduce the data is necessary in order to confirm the result.

Following oral administration of the probiotic compositions for 4 weeks (Table 5), the mice were given 5 mg/mL IS dissolved in 0.5% methyl cellulose by tube feeding. Two hrs later, the blood IS levels of these mice that received bacteria are all lower than the control groups that received no treatment, and the Kefir group shows the lowest IS level. This result is not consistent with the result obtained from the toxin-challenged groups (Fig. 1).

**Table 5 Blood indoxyl sulfate (IS) levels at 2 hrs post IS treatment**

| following oral administration of bacteria for 4 weeks. | |
|---|---|
| Sample | Indoxyl sulfate_concentration (ug/mL) |
| Control | 298.7423 |
| Kefir | 252.8100 |
| M1 | 260.5693 |
| 10⁷ | 264.1978 |
| 10⁸ | 263.9259 |

### 3. Conclusion:

The results indicates that all probiotic compositions can reduce IS levels in mice, but the results are not consistent between mice with or without IS treatment. Oral feeding IS might not be a good animal model for testing IS clearance efficiency. Moreover, additional positive control group may be necessary for further comparison. Urine and blood collections are also rather restricted in mice. Thus, rats with Cisplatin-induced acute kidney injury are used instead in the following testing to repeat the experiment with inclusion of urine analysis and additional positive control groups.

### Example 3

### 1. To provide a probiotic composition, Pm-1, for clearing uremic toxins:

(1) The composition comprises *Lactobacillus plantarum* BCRC 12251, *Lactobacillus paracasei* BCRC 12188, *Streptococcus thermophilus* BCRC 13869 ;
(2) Said composition further comprises pharmaceutically acceptable vehicles, excipients, diluents and adjuvants;
(3) The concentration of various probiotics in Pm-1 is 10⁹ CFU/ mL;
(4) The ratios of various probiotics in Pm-1 are 40∼60%, 20∼30 % and 20∼30% for *Lactobacillus plantarum* BCRC 12251, *Lactobacillus paracasei* BCRC 12188, *Streptococcus thermophilus* BCRC 13869, respectively.
(5) Said Pm-1 probiotic composition can be used for reducing the protein-bound uremic toxins in blood.

### 2. The effects of probiotics on uremic toxin clearance

### (1) The effect of a single strain on indoxyl sulfate (IS) clearance:

An aliquot of 100 µL sample were collected from 12 different activated bacterial cultures (wherein strains 12251 and 12188 are the strains exhibit better clearance effects in the Example 1) and then inoculated into culture media containing different uremic toxins. The inoculated cultures were centrifuged at 12,000 rpm for 5 min at 0 hr, 48hrs and 96 hrs post inoculation, and the supernatant were then subjected to filtration using a 0.22µm filter membrane (Pall, NY, USA). MRS culture media containing indoxyl sulfate but without probiotic bacteria and plain MRS were used as the positive and negative (NC) controls, respectively.

Next, the collected samples were analyzed by HPLC and then converted into percentages as shown in Table 6. The concentrations obtained at 0 hr is used as the initial concentrations for calculation of clearance rates at 48hr and 96 hr. The results indicate that strains EF, 12188, 12251 and 13869 have better clearance rates for indoxyl sulfate.

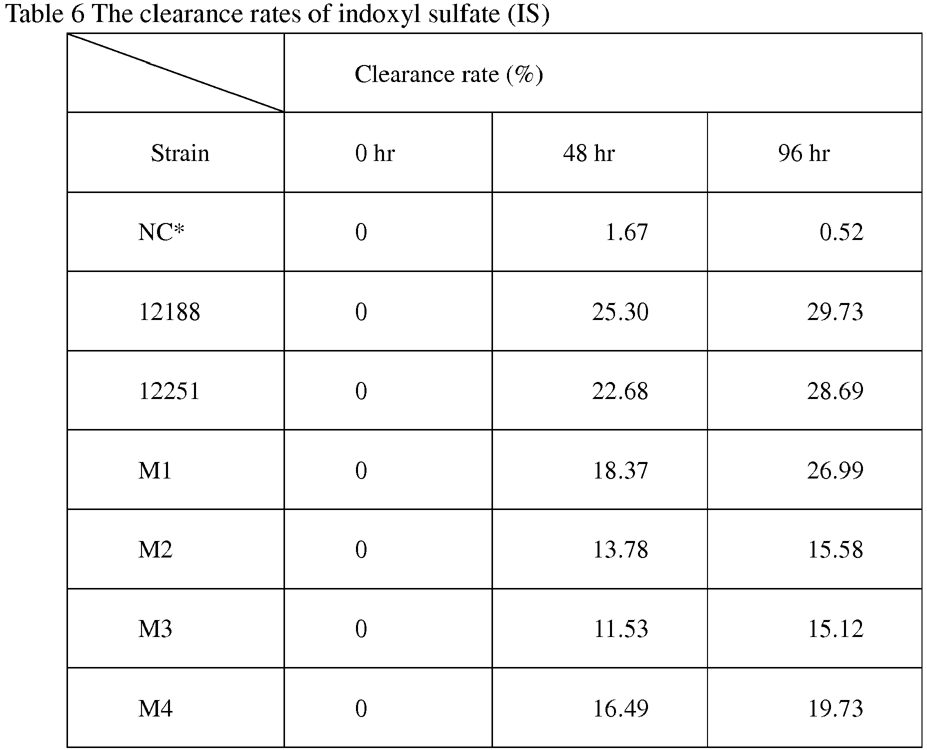

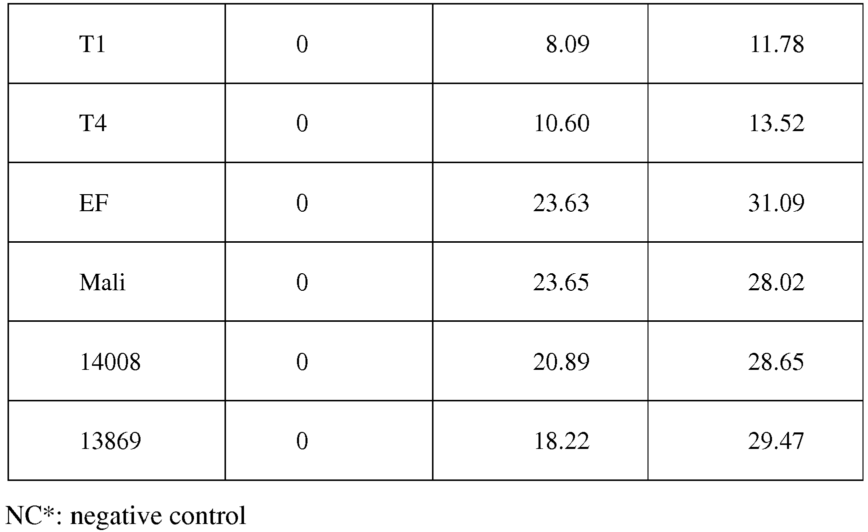

### (2) The effects of mixed strains on indoxyl sulfate clearance:

Figure 2 shows the IS clearance rates of the single strains 12188, 12251, 13869 and EF, and mixed strains 12251+12188, 12251+EF, 12188+EF and 12251+12188+13869, wherein the concentrations at 0 hr were used as the initial concentrations for calculations of the clearance rates at 48 hr and 96 hr. The results suggested that 12251+12188+13869 and 12251+EF have the best clearance effects on IS.

### Example 4

### Cisplatin-induced acute kidney injury in rats

### 1.Probiotic compositions:

The two probiotic compositions with better IS clearance effect were selected to treat Cisplatin-induced kidney injury in rats so as to examine their effects. As shown in Fig. 7, the probiotic compositions that have better IS clearance effect are probiotic mix-1 (Pm-1) and probiotic mix-2 (Pm-2) according to prior *in vitro* testing.

**Table 7 Mixed probiotic compositions:**

| Strain of bacteria | Source |
|---|---|
| Probiotic mix-1 (Pm-1) | |
| *Lactobacillus plantarum* | BCRC 12251 |
| *Lactobacillus paracasei* | BCRC 12188 |
| *Streptococcus thermophilus* | BCRC 13869 |

| Probiotic mix-2 (Pm-2) | |
|---|---|
| *Enterococcus faecalis* | Lab kefir |
| *Lactobacillus plantarum* | BCRC 12251 |

### 2.Animal study:

Male SD rats at the age of 14 weeks were orally administered with the mixed probiotic compositions for 5 days continuously, and Cisplatin (10 mg/kg of the rat body weight) was intraperitoneally (i.p.) injected on day 2 following oral administration to induce kidney damage. The blood samples were collected every two days and examined for IS concentrations and kidney biomedical parameters. Four days after i.p. injection, the kidneys were separated from the rats for histological sections and IS concentrations in various organs was also determined.
A total of five groups were included:
(1) control group: rats received only PBS (phosphate buffer
   saline) injection, no Cisplatin;
(2) positive control group: rats received Cisplatin injection
   without administration of the probiotic composition;
(3) test group 1: rats received Cisplatin injection and orally
   administrated with Pm-1 probiotic composition (strains 12251+12188+13869);
(4) test group 2: rats received Cisplatin injection and orally
   administrated with Pm-2 probiotic composition (strains 12251+EF);
(5) Wk (Wakamoto) group: rats received Cisplatin injection and
   orally administrated with Wk (Wakamoto) tablets.

For detailed animal study design, please see Fig. 3.

### 3.Results:

### (1) Body weight change (as shown in Fig. 4)

On day 4 of the experiment, the body weights of the rats in the control group received PBS show no significant change (98.43± 6.39%), whereas the weights of the rats in the test group received Cisplatin reduced dramatically (88.33±4.77%, p<0.01). In addition, the body weight lost due to Cisplatin injection cannot be recovered in the rats received Wk tablet (86.39±4.23%), Pm-1 (86.39±8.98%), or Pm-2(86.65±2.27%).

### (2) IS concentrations in serum and urine (Fig. 5)

The plasma IS levels of Cisplatin-injected rats are as high as 395.51±184.55 µg/mL, which indicate the animal model for acute kidney damage was well established. According to the results, high IS levels induced by acute kidney damage can be significantly reduced only in rats treated with Pm-1 probiotic composition (130.69±68.07 µg/mL, p<0.05). Although the plasma IS levels are also slightly reduced in both groups administered with Wk tablet and Pm-2 probiotic composition, no statistical differences were found.

In comparison with normal mice, indoxyl sulfate in urine of rats with acute kidney damage show no significant increase. Yet, interestingly, the indoxyl sulfate levels in urine collected from the rats treated with Wk tablets and Pm-1 probiotic composition are both higher than either control or positive control groups. Nonetheless, no statistically significant differences were found due to substantial variations between individual rat. As for the rats treated with Pm-2 probiotic composition, no effects on urine IS levels were observed.

### (3) IS levels in various organs (Fig. 6):

The IS levels in the kidneys and livers obtained from rats with Cisplatin-induced acute kidney injury are notably higher than the control group (p<0.05). Although orally administered with Pm-1 and Pm-2 can reduce the
accumulation of IS induced by acute kidney injury in the kidneys and liver, no significant differences were found when compared with the positive control group due to substantial variations between rats and high standard deviation.

### (4) Analysis of blood biochemical parameters (Fig.7):

### (a) Blood urea nitrogen (Fig. 7A)

Clinically, blood urea nitrogen (BUN) is one of the most common indicators for analyzing renal function. BUN is accumulated in vivo if the kidneys were damaged and lost their toxin removal functions. In the present example, Cisplatin treatment leads to dramatic increase of BUN levels in rats with acute kidney damage (177.75±34.06 mg/dL; p<0.005); but raised blood urea nitrogen levels in rats with acute kidney damage were not reduced by administrations of Pm-1, Pm-2 and Wk.

### (b) Creatinine (Fig. 7B)

Creatinine is also one of the most common indicator in clinical for analyzing kidney function, and its level is associated with the damage in glomerular cells of the kidneys. In this example, Cisplatin treatment leads to dramatic increase of creatinine levels in rats with acute kidney damage (4.45±1.16 mg/dL; p<0.01); but raised creatinine levels in rats with acute kidney damage were not reduced by administrations of Pm-1, Pm-2 and Wk.

### (c) Uric acid (Fig. 7C)

Kidney malfunctions may result in accumulation of uric acid *in vivo;* and in this example, no significant differences were observed amongst all treatment groups (p>0.05).

### (d) Calcium (Fig. 7D)

Impaired electrolyte regulation of the kidneys may sometimes cause hypocalcemia ; however, according to the animal model for inducing acute kidney damage presented in present example, no hypocalcemia was found. On the contrary, the concentration of blood calcium increases considerably (p<0.05), which indicates that injection of Cisplatin may result in altered electrolyte regulation in the kidneys without causing hypocalcemia. In addition, administrations with Pm-1 and Pm-2 probiotic compositions in the test groups reduced elevated calcium levels caused by acute kidney damage, and Pm-2 significantly reduced blood calcium concentrations induced by acute kidney damage (p<0.05).

### (e) Magnesium (Fig. 7E)

In clinical, polyuria is a commonly experienced symptom in the patients with acute kidney failure when recovery and often leads to hypomagnesemia. In this example, nohypomagnesemia was observed following injection of Cisplatin, which suggests the rats have not yet entered the polyuria stage upon acute kidney damage; moreover, no significant differences were discovered among all treatment groups except the rats administered with Wk tablets exhibiting higher blood magnesium levels, but the differences are not statistically significant (p>0.05).

### (f) Blood ammonia (Fig. 7F)

Clinically, elevated blood ammonia level is mainly associated with liver and metabolic diseases; in contrast, low blood ammonia level is correlated with hypertension and certain medication drugs. In this example, Cisplatin induced low blood ammonia; however, administrations of Wk tablets (p<0.05) and Pm-1 (p<0.01) composition notably reversed the reduced blood ammonia levels, whereas no significant differences were observed in rats administered with Pm-2 probiotic composition.

### (5) Urine analysis (Fig. 8)

### (a) Urine bilirubin

Detectable bilirubin in urine indicates liver disease, bile duct obstruction, and severe kidney glomerular damage. In the example, no bilirubin was detected in the urine samples of the control and all treatment groups, which suggests both the liver and the bile duct of all treatment groups are not damaged.

### (b) Urobilinogen

Elevated urobilinogen level implies liver disease or bile ductobstruction. In present example, the urobilinogen levels of the control and all treatment groups are within the normal range, which indicates both the liver and the bile duct of all treatment groups are not damaged.

### (c) Nitrite

In urinary infection, nitrates is reduced to nitrite salts by bacteria. In the example, no nitrite salts are detected in either the control or the treatment groups. Hence, no urinary infections in rats of any treatment group.

### (d) Urine glucose (Fig 8A)

Damage in the kidneys may increase glucose levels in urine. As shown in Fig. 8A, Cisplatin-induced acute kidney toxicity in rats increases glucose levels in urine; nonetheless, neither Wk nor probiotic compositions administrations can reduce the elevated glucose levels.

### (e) Urine proteins (Fig. 8B)

Kidney damage, especially glomerular or tubular damage or diseases, will increase protein levels in urine as indicated in Fig. 8B that Cisplatin increases the protein levels in urine. In addition, oral administrations of Wk tablets or probiotic compositions are unable to reduce the elevated protein levels in urine.

### (f) The pH (Fig. 8C)

In clinical, diet or kidney inflammation may reduce the pH value of urine. In this example, Cisplatin reduces the pH of urine, which is then recovered by administration with Pm-2 probiotic composition. Because the decrease of pH value in urine shows no statistical differences. As for other treatment groups, no effects are found.

### (g) Urine specific gravity (Fig. 8D)

Decreased urine specific gravity may be due to kidney diseases, impaired renal medulla, diabetes insipitus and excess water intake, and increased urine SG may be resulted from dehydration or diuretics treatment. However, in present example, no differences are observed between the control and all the treatment groups.

### (h)Urine blood (Fig. 8E)

Kidney and urinary system diseases or inflammation may result in detection of blood in urine. In this example, administrations of Wk tablets and Pm-2 probiotic composition considerably reduced the hematuria induced by Cisplatin (p<0.05).

### (i) Leukocytes(Fig. 8F)

The cause of leukocytes in urine may be due to infections in the kidney or urinary systems, or kidney medulla damage triggered by toxins. According to the example, leukocytes increase induced by Cisplatin cannot be reduced by administrations of either Wk tablets or probiotic compositions.

### (j) Ketone (Fig. 8G)

Clinically, elevated ketone in urine may be due to prolonged abstention, alcoholic or diabetic ketoacidosis, hyperthyroidism and fever. In the example, Cisplatin raises the ketone level in urine, which is reversed by administrations of Wk tablets and Pm-2 probiotic composition, but no statistical differences are found. Additionally, no effects are observed in other treatment groups.

### 4. Conclusion:

### (1) Cisplatin:

Kidney damage induced by peritoneal injection of Cisplatin dramatically reduces the body weights of rats; considerably increases IS levels in blood, kidney and liver; and notably lowers blood urea nitrogen, creatinine and uric acid levels as well as blood nitrates. Furthermore, Cisplatin injection also remarkably increases glucose, protein, WBC, and ketone bodies levels in urine as well as the pH and hematouria.

### (2) Pm-1 probiotic composition:

The Pm-1 probiotic mix significantly reduces elevated blood IS level in the Cisplatin-treated rats, increases removal of urine IS as well as decreases IS levels in the kidneys and livers. Moreover, it can also reverse low blood ammonia caused by Cisplatin and reduce blood calcium elevation resulted from acute kidney injury.

### (3) Pm-2 probiotic composition:

Although Pm-2 probiotic composition cannot reduce elevated blood IS levels in the Cisplatin-treated rats, the results nonetheless demonstrates the decrease of IS levels in the kidneys and liver as well as dramatic reduction of hematuria caused by kidney damage. In addition, the Pm-2 composition may also reverse ketone body increase resulted from injection of Cisplatin, while considerably reduce elevated blood calcium caused by acute kidney damage.

It can be concluded from the above examples that the Pm-1 probiotic composition of the present invention can effectively clear the uremic toxins. Most particularly, these uremic toxins are protein-bound uremic toxins, such as indoxyl sulfate, p-cresyl sulfate and phenol. Additionally, Pm-1 can efficiently reduce the rise of blood indoxyl sulfate induced by kidney damage, increase elimination of indoxyl sulfate, as well as decrease IS concentrations in the kidneys and livers. In addition to the abovementioned advantages, said Pm-1 probiotic composition is not only easy to prepare and use, but also is very cost-effective and highly economic when compared with other traditional methods for clearing uremic toxins.

The foregoing examples and embodiments are merely better examples of the present invention; therefore, it should be understood that they are only for illustration purpose and shall not limit the scope of the present invention. Any variations or modifications made according to the claims of the present invention are remained within the scope of the present invention.

In summary, present invention provides a probiotic composition and its applications thereof for clearing uremic toxins. Hence, the invention meets the requirements of novelty and inventive step as well as provides multiple advantages that are not found in other known compositions for clearing uremic toxins.

## Claims

1. A probiotic composition for the treatment of uremic toxins, wherein the probiotic composition comprises at least one selected from the group consisting of: Lactobacillus plantarum BCRC 12251, Lactobacillus paracasei BCRC 12188, Streptococcus thermophilus BCRC 13869 and pharmaceutically acceptable vehicles, excipients, diluents and adjuvants.

2. The probiotic composition of claim 1, wherein said composition comprises Lactobacillus plantarum BCRC 12251, Lactobacillus paracasei BCRC 12188, Streptococcus thermophilus BCRC 13869 and pharmaceutically acceptable vehicles, excipients, diluents and adjuvants.

3. The probiotic composition of any one of claims 1 and 2, wherein the uremic toxins are protein-bound uremic toxins.

4. The probiotic composition of any one of claims 1 to 3, wherein the protein-bound uremic toxins are indoxyl sulfate, p-cresol and phenol.

5. The probiotic composition of any one of claims 2 to 4, wherein the concentrations of the probiotics are 10⁷-10¹⁰ CFU/mL.

6. The probiotic composition of any one of claims 2 to 4, wherein the amounts of *Lactobacillus plantarum* BCRC 12251, *Lactobacillus paracasei* BCRC 12188, and *Streptococcus thermophilus* BCBR 13869 are 40-60%, 20-30% and 20-30%, respectively.
